# EUROPEAN PATENT APPLICATION

(11) **EP 3 153 853 A1**
(43) Date of publication of application: **12.04.2017**
(21) Application number: 15803252.4
(22) Date of filing: 14.05.2015
(51) Int. Cl.: G01N 33/00, G01N 33/48, B81B 1/00, B81C 1/00

(54) **PLASTIC MICROCHIP**

(30) Priority: 03.06.2014 KR 20140067508
(71) Applicant: Nanoentek Inc., Seoul 152-740 (KR)
(72) Inventor: KIM, Yu-Rae, Hwaseong-si Gyeonggi-do 445-926 (KR); KIM, Jae-Jeong, Hwaseong-si Gyeonggi-do 445-926 (KR); HUR, Dae-Sung, Suwon-si Gyeonggi-do 440-330 (KR)
(74) Representative: advotec.
(86) International application number: PCT/KR2015/004865
(87) International publication number: WO 2015/186913

(57) **Abstract**

A plastic microchip is disclosed. The plastic microchip according to examples of the present invention enables a bonding material to be quickly and uniformly diffused along a bonding surface so as to accurately and easily bond an upper substrate and a lower substrate, maintain a shape of a bonding part when separating an injection-molded article from a mold so as to minimize a design error of a channel height, and prevent the bonding material from leaking inside a space of a bonding jig or a sample charging part, thereby improving product productivity and reliability.

## Description

### TECHNICAL FIELD

The present invention relates to a plastic microchip, and more particularly, to a plastic microchip in which a bonding material diffuses quickly and uniformly along an bonding interface between an upper substrate and a lower substrate to precisely bond the upper and lower substrates to each other, and is prevented from leaking into a bonding jig or a sample filling space, thereby improving product productivity and reliability.

### BACKGROUND ART

In general, a fluid sample analysis has been widely used in the field of diagnosis performed by analyzing blood and body fluids collected from a patient and the like, as well as in the field of chemical and biotechnology. Recently, in order to perform such a fluid sample analysis more conveniently and efficiently, various types of small-sized analysis and diagnostic equipment and technologies thereof have been developed.

In particular, lab-on-a-chip technology is technology of performing, on a small-sized chip, various experimental processes performed in a laboratory, e.g., separation, refinement, mixing, and cleaning of a sample, using micro-fluidics technology or the like.

In relation to the lab-on-a-chip technology, a portable DNA test device, for personal identification, through which a process of extracting and analyzing DNA may be performed on a chip at a time has been developed. Besides, thelab-on-a-chip technology has been actively applied in various fields of the industry.

Furthermore, in the field of in vitro diagnostics, research has been actively conducted on portable diagnosis tools through which an individual person can easily perform, on the spot, a complicated thorough examination conducted in a hospital or a laboratory using blood, body fluids, or the like, i.e., the field of point-of-care testing (POCT).

The POCT is technology enabling it to conveniently diagnose a disease at a medical treatment site, such as an emergency room, an operating room, or a general home, and is a field which has increased in necessity and demand, in preparation for an aging society and a welfare society. Although diagnosis tools for measuring blood sugar have recently become the mainstream of the market, the demand for a diagnosis tool for diagnosing various biological materials such as lactic acid, cholesterol, urea, and infectious pathogenic bacterium has also increased quickly as an actual need for the POCT has increased.

In general, in such analysis or diagnosis technologies, whether a fluid and either antibody protein or other various samples fixed inside a chip react with each other is detected and analyzed while moving the various fluid samples through a micro-channel formed in the chip, according to various detection methods.

The lab-on-a-chip technology related to the above detection and analyzing is to perform various experimental processes, which are performed in a laboratory, e.g., sample separation, refinement, mixing, labeling, analysis, cleaning, etc., on a small-sized chip. To design a lab-on-a-chip, techniques related to micro-fluidics and a micro-liquid handling system (micro-LHS) have been mainly used. Furthermore, in relation to manufacture of a chip structure for realizing the micro-fluidics and the micro-LHS, chips having a micro-channel therein using semiconductor circuit design technology have been on the market.

A plastic microchip for use in the POCT or a lab-on-a-chip is formed of polyethylene (PE) derivatives, such as polycarbonate (PC), polystyrene (PS), polypropylene (PP), or polyethylene terephthalate (PET), polymethyl methacrylate (PMMA), etc., or an acryl-based plastic material, and is disposable.

The plastic microchip is manufactured by bonding an upper substrate and a lower substrate to each other. In order to charge a sample, a sample filling space, a micro-structure, or the like is provided to a predetermined height between the upper substrate and the lower substrate bonded to each other.

The plastic microchip should be precisely manufactured such that the sample filling space has a height of several µm to several hundreds of µm. Thus, the plastic microchip may function perfectly when the upper and lower substrates including the sample filling space or the micro-structure are very precisely and exactly bonded to each other. To this end, a technique of quickly and uniformly diffusing a bonding material into parts of the upper and lower substrates to be bonded to each other is needed.

The upper and lower substrates are bonded to each other by pressing the parts thereof, which are to be bonded to each other, by a bonding jig. When the bonding material flows to a pressing surface of the bonding jig during this process, a manufacturing process should be stopped and resumed after the bonding material is cleaned or removed, thereby lowering productivity.

The upper and lower substrates are bonded to each other as the bonding material permeates the parts due to a capillary force during the bonding of the upper and lower substrates. In this case, it is important to control the bonding material to not leak into the sample filling space while securing the directionality and fluidity of the bonding material so that it may quickly flow into the parts of the upper and lower substrates.

Accordingly, in order to solve these problems, there is a growing need to develop a plastic microchip in which, when an upper substrate and a lower substrate are bonded to each other, a bonding material quickly and uniformly diffuses along an bonding interface between the upper and lower substrates to precisely bond the upper and lower substrates to each other and is prevented from leaking into a bonding jig or a sample filling space, thereby enhancing product productivity and reliability.

### DETAILED DESCRIPTION OF THE INVENTION

### TECHNICAL PROBLEM

Embodiments of the present invention are directed to precisely and easily bonding an upper substrate and a lower substrate to each other by quickly and uniformly diffusing a bonding material along an bonding interface between the upper and lower substrates.

Embodiments of the present invention are also directed to maintaining a shape of a bonding part when a molded part is separated from a mold, thereby minimizing a design error of a channel height.

Embodiments of the present invention are also directed to preventing a bonding material from leaking into a bonding jig or a sample filling space, thereby improving product productivity and reliability.

### TECHNICAL SOLUTION

According to an aspect of the present invention, there is provided a ∼

According to another aspect of the present invention, there is provided a ∼

### ADVANTAGEOUS EFFECTS

According to embodiments of the present invention, an upper substrate and a lower substrate may be precisely and easily bonded to each other by quickly and uniformly diffusing a bonding material along a bonding interface between the upper and lower substrates.

Furthermore, a shape of a bonding part may be maintained when a molded part is separated from a mold, thereby minimizing a design error of a channel height.

In addition, the bonding material may be prevented from leaking into a bonding jig or a sample filling space, thereby improving product productivity and reliability.

### DESCRIPTION OF THE DRAWINGS

FIG. 1 is a perspective view of a plastic microchip according to a first embodiment of the present invention.
FIG. 2 is a plan view of an upper substrate and a lower substrate of the plastic microchip according to the first embodiment of the present invention.
FIG. 3 is a cross-sectional view of the plastic microchip according to the first embodiment of the present invention.
FIG. 4 is a partial enlarged view of FIG. 3.
FIG. 5 is a partial enlarged view illustrating a case in which a bonding part extends downward from the upper substrate.
FIG. 6 is a perspective view of a plastic microchip according to a second embodiment of the present invention.
FIG. 7 is a plan view of an upper substrate and a lower substrate of the plastic microchip according to the second embodiment of the present invention.
FIG. 8 is a cross-sectional view of the plastic microchip according to the second embodiment of the present invention.
FIG. 9 is a perspective view of a plastic microchip according to a third embodiment of the present invention.
FIG. 10 is a plan view of an upper substrate and a lower substrate of the plastic microchip according to the third embodiment of the present invention.
FIG. 11 is a cross-sectional view of the plastic microchip according to the third embodiment of the present invention.
FIG. 12 is a cross-sectional view illustrating various modified examples of a plastic chip according to the present invention.

### MODE OF THE INVENTION

Hereinafter, exemplary embodiments of the present invention will be described in detail with reference to the accompanying drawings. The present invention is, however, not limited to the embodiments set forth herein and may be embodied in many different forms. Rather, these embodiments are provided so that this disclosure will be thorough and complete and will fully convey the concept of the invention to those of ordinary skill in the art. In the drawings, the same reference numerals represent the same elements throughout the drawings.

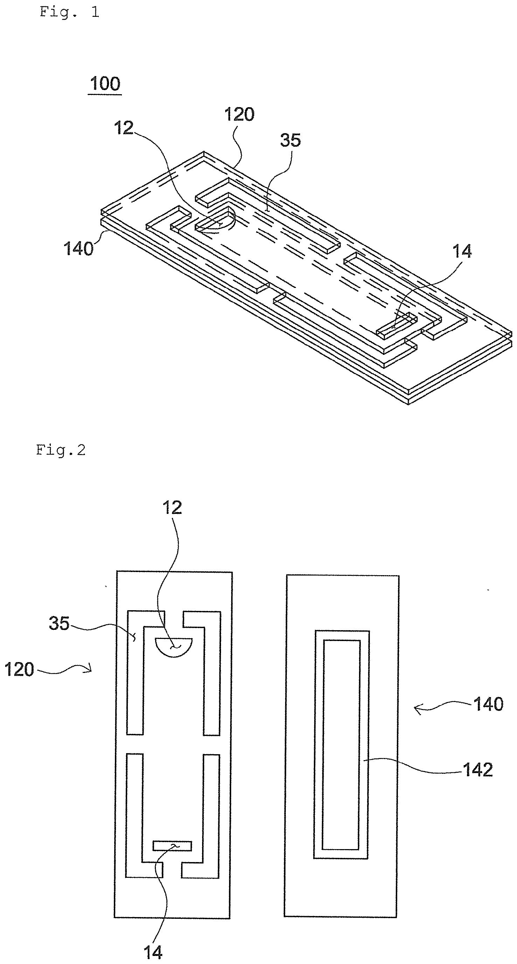

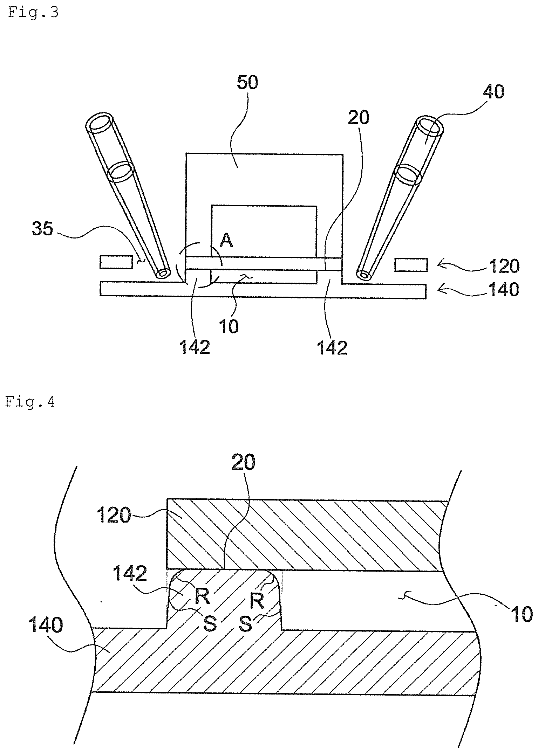

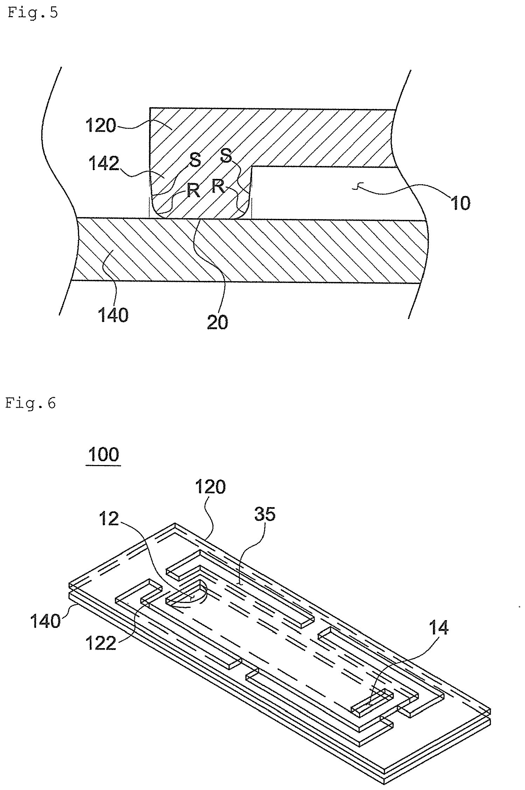

FIG. 1 is a perspective view of a plastic microchip according to a first embodiment of the present invention. FIG. 2 is a plan view of an upper substrate and a lower substrate of the plastic microchip according to the first embodiment of the present invention. FIG. 3 is a cross-sectional view of the plastic microchip according to the first embodiment of the present invention. FIG. 4 is a partial enlarged view of FIG. 3. FIG. 5 is a partial enlarged view illustrating a case in which a bonding part extends downward from the upper substrate.

Referring to FIGS . 1 to 5, a plastic microchip 100 according to the first embodiment of the present invention may largely include a sample filling space 10 formed to a certain height between an upper substrate 120 and a lower substrate 140 bonded to each other; a bonding part 142 extending by a certain length from at least one of the upper substrate 120 and the lower substrate 140, forming sidewalls of the sample filling space 10, and having an end surface forming an bonding interface 20 between the upper substrate 120 and the lower substrate 140; and round parts R formed on corners at a side of the bonding interface 20 of the bonding part 142 and each having a round shape.

The upper and lower substrates 120 and 140 may each have a rectangular flat shape. The sample filling space 10 is formed between the upper and lower substrates 120 and 140 bonded to each other.

The sample filling space 10 may be formed to have a certain width and length between the upper substrate 120 and the lower substrate 140, and may have a rectangular shape similar to the upper substrate 120 and the lower substrate 140.

The sample filling space 10 may have a predetermined height D as in the previous embodiment. In detail, the sample filling space 10 may have a height of several µm to several hundreds of µm. The sample filling space 10 forms a micro-channel through which a sample to be injected flows, and may include a micro-structure such as pillars.

A sample injection port 12 is formed at one side of the sample filling space 10, into which a sample to be tested is injected. A sample discharge port 14 is formed at another side of the sample filling space 10, through which a sample remaining after a test is discharged.

In detail, the sample injection port 12 and the sample discharge port 14 are formed in the upper substrate 120 in the present embodiment but the present invention is not limited thereto. At least one of the sample injection port 12 and the sample discharge port 14 may be formed in the lower substrate 140 if necessary.

The bonding part 142 may extend by a certain length from at least one of the upper substrate 120 and the lower substrate 140, and form the sidewalls of the sample filling space 10. An end surface of the bonding part 142 forms the bonding interface 20 between the upper substrate 120 and the lower substrate 140.

Although the bonding part 142 extends from the lower substrate 140 in the present embodiment, it may extend from the upper substrate 120 or both the upper substrate 120 and the lower substrate 140.

In FIGS. 1 to 4, the bonding part 142 protrudes to a certain height from a top surface of the lower substrate 140. When the upper substrate 120 is bonded to a top surface the bonding part 142, the bonding interface 20 is formed.

Thus, the bonding part 142 forms boundaries of the sample filling space 10, and a height of the sample filling space 10 is determined by the height to which the bonding part 142 protrudes.

The plastic microchip 100 according to the present invention includes the round parts R formed on the corners at a side of the bonding interface 20 of the bonding part 142 and each having a round shape. FIG. 4 is a partial enlarged view of the bonding part 142 of FIG. 3, in which the round parts R formed on upper corners of the bonding part 142 are shown. The round part R may be formed on all corners of the bonding interface 20 or only on outer corners of the bonding interface 20. FIG. 4 illustrates a case in which the round parts R are formed on the corners of the opposite ends of the bonding interface 20.

The round parts R may be applied during design and processing of a mold for injection-molding the upper substrate 120 and the lower substrate 140. However, even if in a state in which a shape of the round part R is not applied to the mold, the round parts R may be naturally formed on corners of the bonding interface 20 when injection-molding is performed in a fine form as in the present invention.

The round parts R may be formed along an outer side of the bonding interface 20 to be consecutively connected to each other. When a bonding material injected near the bonding interface 20 comes in contact with the round parts R, the bonding material may uniformly permeate the bonding interface 20 while quickly diffusing to the outer side of the bonding interface 20 along the consecutive round parts R.

Here, a bonding-material injection port 35 may be formed in the upper substrate 120, through which the bonding material may be injected near the bonding interface 20 between the upper substrate 120 and the lower substrate 140 bonded to each other. As illustrated in FIGS. 1 to 3, the bonding-material injection port 35 may be divided into four parts along the circumference of the bonding part 142 to form a passage or path through which an injector 40 containing the bonding material may access the bonding interface 20.

When the injector 40 is located adjacent to the bonding interface 20 and the bonding material is injected in a state in which the upper and lower substrates 120 and 140 are brought into contact with each other such that surfaces thereof to be bonded coincide, the bonding material injected near the bonding interface 20 comes in contact with the round parts R and permeates the bonding interface 20 due to a capillary force as described above. In this state, the upper and lower substrates 120 and 140 may be bonded to each other by pressing them against each other by a bonding jig 50.

In this case, an operator need not enforcedly inject the bonding material into the bonding interface 20 through the round parts R. The bonding material diffuses into the bonding interface 20 due to a capillary force when a sufficient amount of the bonding material is dropped to positions near the round parts R using the injector 40 so that the bonding material may come in contact with the round parts R.

The bonding material may be injected into two to four positions spaced an appropriate distance from one another. After the bonding material diffuses along the round parts R due to a capillary force, it permeates the bonding interface 20, thereby bonding the upper and lower substrates 120 and 140 to each other. For example, the bonding material may be injected into two positions which are spaced from each other in a diagonal direction of the sample filling space 10 to be symmetrical with each other.

Here, the bonding material employed in the present invention may be an organic solvent which melts the bonding interface 20 between the upper and lower substrates 120 and 140 to bond the upper and lower substrates 120 and 140 to each other.

In detail, the upper and lower substrates 120 and 140 may be formed of polyethylene (PE) derivatives, such as polycarbonate (PC), polystyrene (PS), polypropylene (PP), polyethylene terephthalate (PET), etc., polymethyl methacrylate (PMMA), or an acryl-based plastic material.

The organic solvent may be an arbitrary organic solvent which melts the above materials, e.g., ketone, aromatic hydrocarbon, halogenated hydrocarbon, or a mixture thereof, and preferably, acetone, chloroform, methylene chloride, carbon tetrachloride, or a mixture.

According to an embodiment of the present invention, the plastic microchip 100 may have a slope gradient S in a direction in which the bonding part 142 extends so that the plastic microchip 100 may taper.

The slope gradient S is set such that the plastic microchip 100 tapers in the direction in which the bonding part 142 extends. The direction in which the bonding part 142 extends is opposite to a direction in which a molded part is separated from a mold after injection-molding is performed. The slope gradient S is set so that the upper and lower substrates 120 and 140 may be easily separated from the mold after injection-molding is performed.

As described above, the height of the sample filling space 10 is determined by the height of the bonding part 142. When the bonding part 142 has the slope gradient S, a portion of the bonding part 142 may be prevented from being stuck to a mold when a molded part is separated from the mold. Thus, the upper and lower substrates 120 and 140 may be separated from the mold while designed shapes thereof may be maintained, thereby minimizing a design error of the height of the sample filling space 10.

When the sample filling space 10 of the plastic microchip 100 according to the present invention is actually designed to have a height of 100 µm, an actual molded part may be manufactured within a range of 95 to 105 µm. Accordingly, an error range may be controlled to be 5% or less.

Although it is illustrated in the present embodiment that the slope gradient S is applied only to the bonding part 142, the slope gradient S is applicable to both outer parts and inner vertical parts of the upper substrate 120 and the lower substrate 140.

As illustrated in FIG. 5, the bonding part 142 may extend from the upper substrate 120. In this case, the round parts R and the slope gradient S described above are also applicable in different directions.

In order to manufacture the plastic microchip 100 having the above structure, first, a mold should be manufactured. In a process of manufacturing the mold, first, an actual molded part is designed. Although first design of the actual molded part is completed, design for processing and injecting should be reviewed and modified to precisely shape a molded part which is a micro-unit before the mold is manufactured.

Then, the mold is designed on the basis of design factors modified and reviewed and is thereafter manufactured through N/C processing according to the design of the mold. In this case, a reading surface of the sample filling space 10 is polished, and a corrosion process or a grinding process using sandpaper may be additionally performed.

When primary manufacture of the mold is completed, injection pressure and mold temperature conditions are set, and an injection test is performed. The dimensions of parts of an injection-molded product shaped through the injection test are measured and the parts are tested, and finishing is performed by applying modifications into the mold according to a result of measuring the dimensions of the parts and testing the parts.

When the manufacture of the mold is finally completed, the upper and lower substrates 120 and 140 of the plastic microchip 100 are manufactured by injection-molding and cleaned by air-brushing.

Then, a plasma surface treatment is performed on a surface of the upper substrate 120 and a surface of the lower substrate 140 facing each other when the upper and lower substrates 120 and 140 are bonded to each other. In detail, the surfaces of the upper lower substrates 120 and 140 bonded to each other are modified by emitting O₂ plasma thereto so that they have C=O group. Thus, hydrophobic surfaces of the upper lower substrates 120 and 140 are changed to have hydrophilic properties.

As described above, the surfaces of the upper lower substrates 120 and 140 having the hydrophilic properties allow a sample injected while a contact angle changes to be easily loaded, and the bonding material to well permeate and diffuse into the bonding interface 20.

After the plasma surface treatment is performed, the upper and lower substrates 120 and 140 are put together, and the bonding material is injected therebetween while press is applied to them by the bonding jig 50. In this case, as described above, when the bonding material injected near the bonding interface 20 comes in contact with the round parts R, the bonding material may uniformly permeate the bonding interface 20 while quickly diffusing to an outer side of the bonding interface 20 along the round parts R which are consecutively connected to each other.

It stands by for a certain time until the upper and lower substrates 120 and 140 are bonded to each other while pressure is applied thereto by the bonding jig 50 in the above state. Basically, the pressure applied to the upper and lower substrates 120 and 140 may be, for example, about 0.3 to 0.4 MPa . After the bonding material is injected, it may stand by for 6 to 12 seconds under the above pressure, thereby completing the bonding of the upper substrate 120 and the lower substrate 140 to each other.

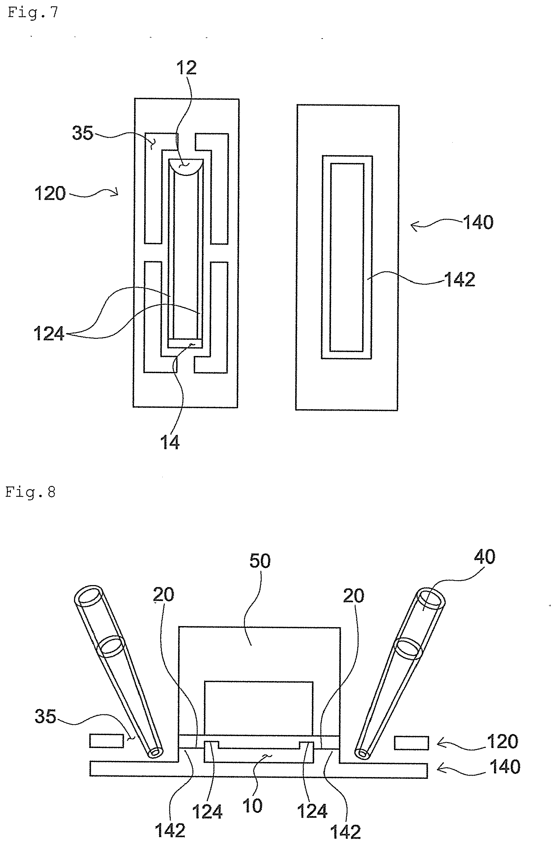

FIG. 6 is a perspective view of a plastic microchip according to a second embodiment of the present invention. FIG. 7 is a plan view of an upper substrate and a lower substrate of the plastic microchip according to the second embodiment of the present invention. FIG. 8 is a cross-sectional view of the plastic microchip according to the second embodiment of the present invention.

Referring to FIGS. 6 to 8, a plasticmicrochip 100 according to the second embodiment of the present invention may include an inflow-prevention channel 124 formed to be sunken in adjacent to an bonding interface 20 inside a sample filling space 10 to prevent a bonding material from flowing from the bonding interface 20 to the sample filling space 10.

Although not particularly shown in FIGS. 6 to 8, the round parts R and the slope gradient S described above in the previous embodiment maybe also applied to the plastic microchip 100 according to the second embodiment. This may also apply to a third embodiment to be described below and modified examples thereof.

As described above, when the bonding material is injected, it diffuses along the bonding interface 20 due to a capillary force. In this case, when a portion of the bonding material leaks into the sample filling space 10 and is then hardened, unintended irregularities may be formed on an internal side surface of the sample filling space 10. The irregularities may affect a test result when a product is used, thereby lowering the precision of the product.

However, when the inflow-prevention channel 124 is provided, the bonding material may permeate only the bonding interface 20 without leaking into the sample filling space 10. Thus, the sample filling space 10 may be formed as originally intended, thereby increasing the precision of a test.

As illustrate FIGS. 6 and 7, the inflow-prevention channel 124 may be formed at a position adjacent to the bonding interface 20 between a sample injection port 12 and a sample discharge port 14 to be long in a direction perpendicular to the sample injection port 12 and the sample discharge port 14. Furthermore, the inflow-prevention channel 124 may be sunken to a certain depth on an upper substrate 120 along inner boundary lines of the bonding interface 20, thereby forming a step. Accordingly, the inflow-prevention channel 124 may prevent the bonding material from flowing into the sample filling space 10.

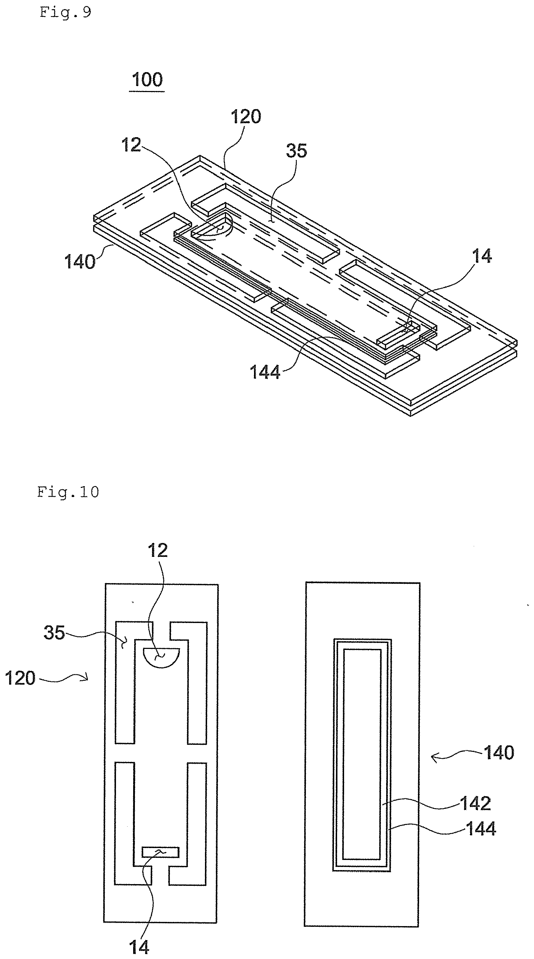

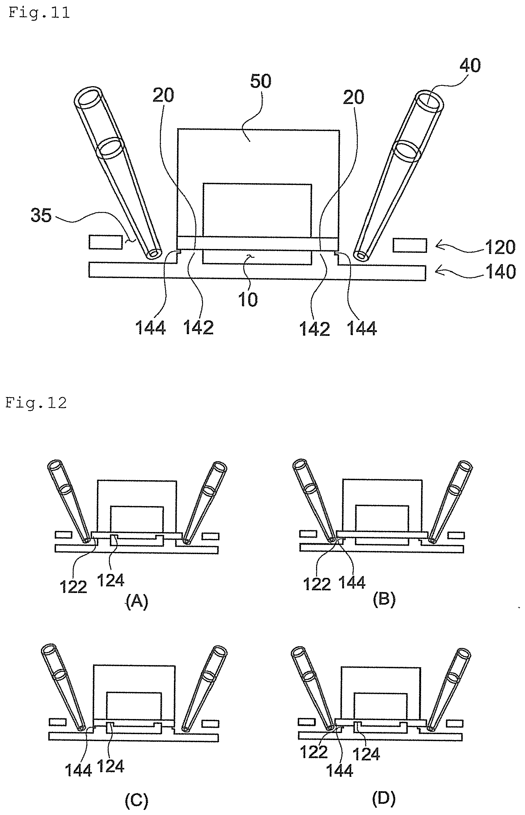

FIG. 9 is a perspective view of a plastic microchip according to a third embodiment of the present invention. FIG. 10 is a plan view of an upper substrate and a lower substrate of the plastic microchip according to the third embodiment of the present invention. FIG. 11 is a cross-sectional view of the plastic microchip according to the third embodiment of the present invention.

Referring to FIGS. 9 to 11, a plastic microchip 100 according to the third embodiment of the present inventionmay include a micro-distribution channel 144 formed along an outer side of a bonding part 142 and forming a flow channel through which a bonding material may quickly move along the circumference of a bonding interface 20.

As described above, when during a bonding process, the bonding material permeates and diffuses into the bonding interface 20 due to a capillary force to bond an upper substrate 120 and a lower substrate 140 to each other. In this case, the directionality and fluidity of the bonding material should be secured so that the bonding material may quickly flow along the bonding interface 20.

The micro-distribution channel 144 additionally provides a path in which the bonding material may quickly flow along the circumference of the bonding interface 20, in addition to the round parts R described above in the first embodiment. Thus, the injected bonding material may quickly flow and diffuse along the micro-distribution channel 144 and then permeate the bonding interface 20 via the round parts R. Thus, the micro-distribution channel 144 causes the bonding material to uniformly diffuse into the entire bonding interface 20.

Accordingly, as the bonding material uniformly diffuses the bonding interface 20 through the micro-distribution channel 144, the upper and lower substrates 120 and 140 may be stably bonded to each other, thereby increasing adhering efficiency and securing the reliability of a product.

As illustrated in FIGS. 9 to 11, the micro-distribution channel 144 may be formed by forming steps by cutting outer parts of the bonding part 142, and induce the bonding material to be quickly applied.

For example, when a sample filling space 10 has a height of about 10 to 20 µm, the micro-distribution channel 144 may be formed to have steps of about 2 to 5 µm. However, the present invention is not limited thereto, and the micro-distribution channel 144 may be formed to have various-sized steps according to the height of the sample filling space 10.

FIG. 12 is a cross-sectional view illustrating various modified examples of a plastic chip according to the present invention.

Referring to FIGS. 11 and 12, the plastic microchip 100 according to the present invention may include an extended part 122 extending by a certain length from an outer side of the bonding interface 20 of the upper substrate 120 to prevent the bonding material from flowing to the bonding jig 50 configured to press the upper substrate 120 against the lower substrate 140 to be bonded to the lower substrate 140, as illustrated in FIG. 12(A), (B), and (D).

As described above, when the upper substrate 120 and the lower substrate 140 are bonded to each other, they are bonded to each other by pressing an upper part of the bonding interface 20 by the bonding jig 50. In this process, when the bonding material flows into a pressing surface of the bonding jig 50, a manufacturing process should be stopped and resumed after the bonding material is cleaned or moved.

However, when the extended part 122 is provided, when the upper substrate 120 and the lower substrate 140 are pressed against each other, the bonding material maybe prevented from flowing to the bonding jig 50. Therefore, the pressing surf ace of the bonding jig 50 may be maintained in a cleaned state and the manufacturing process may be continuously performed without being stopped, thereby increasing productivity.

In the present embodiment, since the bonding-material injection port 35 is formed along the circumference of the bonding part 142, i.e., the circumference of the bonding interface 20, the extended part 122 may be formed to extend along the bonding-material injection port 35.

That is, the extended part 122 extends along the entire circumference of the bonding interface 20 of the upper substrate 120 toward the bonding-material injection port 35, thereby providing a baffle blocking a path in which the bonding material may flow to the bonding jig 50.

In a plastic microchip according to an embodiment of the present invention, the extended part 122, the inflow-prevention channel 124, and the micro-distribution channel 144 described above may be selectively provided together.

That is, the extended part 122 and the inflow-prevention channel 124 may be provided together as illustrated in FIG. 12(A), and the extended part 122 and the micro-distribution channel 144 may be provided together as illustrated in FIG. 12(B).

Furthermore, the inflow-prevention channel 124 and the micro-distribution channel 144 may be provided together as illustrated in FIG. 12(C). All the extended part 122, the inflow-prevention channel 124, and the micro-distribution channel 144 may be provided as illustrated in FIG. 12(D).

In addition, as described above, the extended part 122, the inflow-prevention channel 124, and the micro-distribution channel 144 may be provided independently from or together with the round parts R and the slope gradient S described above in the first embodiment.

In plastic microchips according to the above embodiments of the present invention, a bondingmaterial may quickly and uniformly diffuse along a bonding interface between an upper substrate and a lower substrate to precisely and easily bond the upper substrate and the lower substrate to each other. Furthermore, a shape of a bonding part may be maintained when a molded part is separated from a mold, thereby minimizing a design error of a channel height. In addition, the bonding material may be prevented from leaking into a bonding jig or a sample filling space, thereby improving product productivity and reliability.

While the present invention has been particularly shown and described with reference to exemplary embodiments thereof, it will be understood by those of ordinary skill in the art that various changes in form and details may be made therein without departing from the spirit and scope of the invention as defined by the appended claims. Thus, when modified examples basically include the elements defined in the claims of the present application, they should be understood as falling within the scope of the invention.

## Claims

1. A plastic microchip comprising:
a sample filling space formed to a predetermined height between an upper substrate and a lower substrate bonded to each other;
a bonding part extending by a predetermined length from at least one of the upper substrate and the lower substrate to form sidewalls of the sample filling space, and having an end surface forming an bonding interface between the upper substrate and the lower substrate;
round parts formed on corners at a side of the bonding interface of the bonding part, and having a round shape; and
an inflow-prevention channel sunken in an inner side of the sample filling space, and configured to prevent a bonding material from flowing from the bonding interface to the sample filling space.

2. The plastic microchip of claim 1, wherein the bonding part has a slope gradient to taper in a direction in which the bonding part extends.

3. The plastic microchip of claim 1, further comprising:
a sample injection port formed at one side of the sample filling space; and
a sample discharge port formed at another side of the sample filling space.

4. The plastic microchip of claim 1, further comprising at least one bonding-material injection port formed near the sample filling space, and configured to form a path or passage through which an injector containing the bonding material accesses the bonding interface to bond the upper substrate and the lower substrate to each other.

5. The plastic microchip of claim 4, wherein the bonding material injected near the bonding interface from the injector permeates the bonding interface due to a capillary force.

6. The plastic microchip of claim 1, further comprising an extended part extending by a predetermined length to an outer side of a surface of the upper substrate to be bonded to a surface of the lower substrate, and configured to prevent the bonding material from flowing to a bonding jig, wherein the bonding jig presses the upper substrate against the lower substrate to bond the upper substrate to the lower substrate.

7. The plastic microchip of claim 1, further comprising a micro-distribution channel formed along an outer side of the bonding part, and configured to form a flow channel through which the bonding material quickly moves along circumference of the bonding interface.

8. A plastic microchip comprising:
a sample filling space formed to a predetermined height between an upper substrate and a lower substrate bonded to each other;
a bonding part extending by a predetermined length from at least one of the upper substrate and the lower substrate to form sidewalls of the sample filling space, and having an end surface forming an bonding interface between the upper substrate and the lower substrate;
round parts formed on corners at a side of the bonding interface of the bonding part, and having a round shape; and
a micro-distribution channel formed along an outer side of the bonding part, and configured to form a flow channel through which the bonding material quickly moves along circumference of the bonding interface.

9. The plastic microchip of claim 8, wherein the bonding part has a slope gradient to taper in a direction in which the bonding part extends.

10. The plastic microchip of claim 8, further comprising:
a sample injection port formed at one side of the sample filling space; and
a sample discharge port formed at another side of the sample filling space.

11. The plastic microchip of claim 8, further comprising at least one bonding-material injection port formed near the sample filling space, and configured to form a channel through which an injector containing the bonding material accesses the bonding interface to bond the upper substrate and the lower substrate to each other.

12. The plastic microchip of claim 11, wherein the bonding material injected near the bonding interface from the injector permeates the bonding interface due to a capillary force.

13. The plasticmicrochip of claim 8, further comprising an extended part extending by a predetermined length to an outer side of a surface of the upper substrate to be bonded to a surface of the lower substrate, and configured to prevent the bonding material from flowing to a bonding jig, wherein the bonding jig presses the upper substrate against the lower substrate to bond the upper substrate to the lower substrate.

14. The plastic microchip of claim 8, further comprising an inflow-prevention channel sunken in an inner side of the sample filling space, and configured to prevent a bonding material from flowing from the bonding interface to the sample filling space.
